(19) Europäisches Patentamt
European Patent Office
Office européen des brevets

(11) Publication number: **0 060 028**
**B1**

(12) **EUROPEAN PATENT SPECIFICATION**

(45) Date of publication of patent specification: **04.12.85**

(51) Int. Cl.$^4$: **C 07 D 501/46**

(21) Application number: **82300650.7**

(22) Date of filing: **10.02.82**

(54) **Cephalosporin derivatives and process for their preparation.**

(30) Priority: **02.03.81 JP 29541/81**

(43) Date of publication of application:
**15.09.82 Bulletin 82/37**

(45) Publication of the grant of the patent:
**04.12.85 Bulletin 85/49**

(84) Designated Contracting States:
**CH DE FR GB LI**

(56) References cited:
**EP-A-0 019 067**
**FR-A-2 394 550**

**DERWENT JAPANESE PATENT REPORTS, vol. C, no. 30, 3rd September 1980;**

(73) Proprietor: **AJINOMOTO CO., INC.**
**5-8, Kyobashi 1-chome, Chuo-ku**
**Tokyo 104 (JP)**

(72) Inventor: **Yasuda, Naohiko**
**No. 2-36-2 Hairando**
**Yokosuka-shi Kanagawa-ken (JP)**
Inventor: **Irie, Yasuo**
**No. 958 Kashimada Saiwai-ku**
**Kawasaki-shi Kanagawa-ken (JP)**
Inventor: **Saito, Hideomi**
**No. 143 Aihara**
**Sagamihara-shi Kanagawa-ken (JP)**
Inventor: **Iwagami, Hisao**
**No. 2-20-8, Kannon Kawasaki-ku**
**Kawsaki-shi Kanagawa-ken (JP)**
Inventor: **Nakanishi, Eiji**
**No. 2-20-8 Kannon Kawasaki-ku**
**Kawasaki-shi Kanagawa-ken (JP)**

(74) Representative: **Bond, Bentley George et al**
**HASELTINE LAKE & CO. Hazlitt House 28**
**Southampton Buildings Chancery Lane**
**London, WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 060 028 B1

**Description**

This invention relates to a process for producing imidazole dicarboxylic acid derivatives of the type which may be used as therapeutic agents for humans and animals in the treatment of infectious diseases caused by *Pseudomonas aeruginosa.* It has been discovered that imidazole dicarboxylic acid derivatives represented by the following general formula:

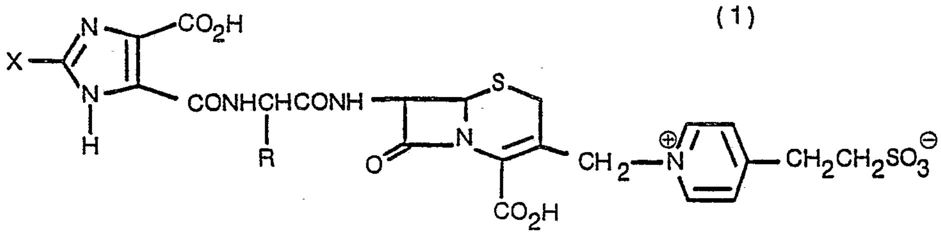

(1)

wherein X and R are hydrogen atoms or substituents (preferred examples of which are indicated below), demonstrate antibacterial property, particularly against *Pseudomonas aeruginosa* and may be used as antibiotics as is disclosed in the specification of laid open Japanese Patent Application No. 55—76887.

The amino acid moiety of the imidazole dicarboxylic acid derivatives of formula 1, i.e. the amino acid moiety containing the radical R, may be, for instance, phenylglycine or 4-hydroxyphenylglycine, and it may be used in the L-form, D-form or DL-form. In many cases, the D-form is preferred because of its antibacterial property.

We have discovered that the derivative wherein one or both of the hydrogen atoms of the carboxyl group carried by the cephalosporin nucleus and imidazole ring is or are replaced by sodium atoms is less toxic than other salts, and we conducted various researches to find a process for the efficient production and separation of the sodium salt, and completed the present invention.

According to the present invention there is provided a process for producing an imidazole dicarboxylic acid derivative of the following general formula in the form of a mono-sodium salt, the di-sodium salt, or a mixture thereof:

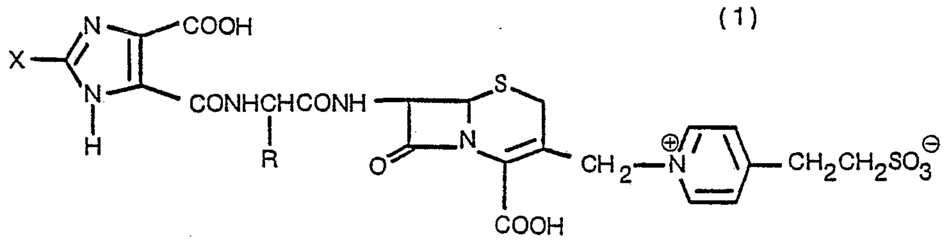

(1)

wherein each of X and R, which may be the same or different, is a hydrogen atom or a substituent; which process comprises reacting, in an aqueous solution containing sodium iodide and sodium hydroxide, 4-pyridineethanesulphonic acid and a compound of the following general formula:—

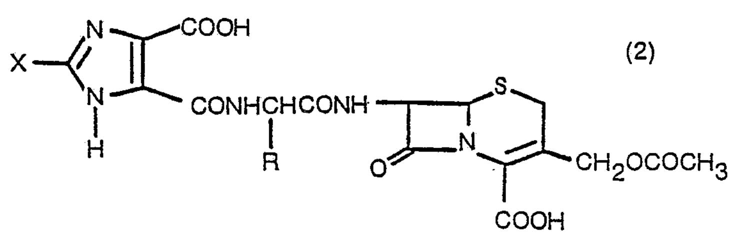

(2)

wherein X and R are as defined above; contacting the reaction solution with a hydrophilic organic solvent which can dissolve sodium iodide, said hydrophilic organic solvent comprising at least one solvent selected from alcohols containing 1 to 4 carbon atoms and acetone, thereby precipitating the said sodium salt(s) of the imidazole dicarboxylic acid derivative; and separating the said precipitated sodium salt(s).

Preferably, X is a hydrogen atom, a halogen atom, a hydroxy group, a hydrocarbyloxy radical, a mercapto group, a hydrocarbylthio radical, a hydrocarbyl sulphonyl or sulphinyl radical, an amino group, a hydrocarbyl amino radical, an acylamino group, a sulphonic acid group, a nitro group, a hydrocarbyl radical or a heterocyclic radical, with any of the hydrocarbyl and heterocyclic moieties being optionally substituted; and R is a hydrogen atom, or a hydrocarbyl or heterocyclic radical which is optionally substituted. Thus, X may be, for instance, a hydrogen atom, a halogen atom, a hydroxyl group, an alkyl-, aralkyl- or aryloxy group, a mercapto group, an alkyl-, aralkyl- or arylthio group, an alkyl-, aralkyl- or

arylsulphonyl or sulphinyl group, an amino group, a mono- or dialkyl-, aralkyl- or arylamino group, an acylamino group such as acetylamino, a sulphonic acid group, a nitro group, a hydrocarbyl radical such as alkyl group, aralkyl group or aryl group, or a heterocyclic group. All of the above-mentioned hydrocarbyl, alkyl, aralkyl, aryl and heterocyclic groups may be optionally substituted. The radical R may be, for instance, a hydrogen atom or a hydrocarbyl or heterocyclic radical which is optionally substituted. Preferably the radical R is a hydrogen atom or an alkyl, aralkyl, aryl or heterocyclic group which is optionally substituted.

Previously the compound of formula 2 was reacted with the 4-pyridineethanesulphonic acid of formula:

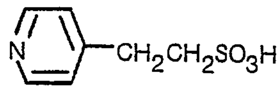

but in the presence of potassium iodide or potassium thiocyanate in order to introduce the pyridinium group, and thereby many products in the form of potassium salts were obtained as disclosed in The Journal of Antibiotics, *29*, 928 (1976). In the above mentioned specification of laid open Japanese Patent Application No. 55—76887, potassium iodide is used in the reaction and the potassium salt obtained thereby then has its potassium ions removed by treating the salt with a cation exchange resin, the acid product then being neutralized by an aqueous solution of sodium hydroxide to convert the same to the sodium salt.

In the above known reaction, it is usual to use a large amount of potassium iodide or potassium thiocyanate as a catalyst, and difficulties were encountered in the recovery of potassium iodide or potassium thiocyanate, on their separation from the desired substance. Conventionally, synthesized adsorbent such as "Amberlite®XAD-2" available from Rohm & Hass Co. was used in a large quantity to remove the salt by chromatography, but this method was quite inferior in that the operation was complicated and the recovery ratio of the desired product was low.

In Example 8 of the EP—A—0019067, there is disclosed a process wherein the disodium salt of 7-β-[D-(−)-α-(4-carboxyimidazole-5-carboxyamido)phenylacetamido]-cephalosporanic acid is reacted with 4-pyridineethanesulphonic acid in the presence of sodium iodide. In this process, also, "Amberlite XAD-2" is used for the separation of the resulting product, namely 7-β-[D-(-)-α-(4-carboxyimidazole-5-carboxamido)-phenylacetamido]-3-(4-α-sulphoethylpyridinium)methyl-3-cephem-4-carboxylic acid disodium salt trihydrate, with the consequent disadvantages regarding yield. In Example 10 of FR—A—2394550, there is disclosed a process similar to that disclosed in Example 8 of EP—A—0019067, except that potassium iodide is used instead of sodium iodide, to obtain the corresponding potassium pentahydrate salt.

The present invention reduces or obviates the difficulties mentioned above and offers an improved process for producing the desired imidazole dicarboxylic acid derivatives.

According to the present invention which uses sodium iodide, the sodium salt is directly obtained, and the step of obtaining the sodium salt after removing the potassium salt (as in the method disclosed in the laid open Japanese Patent Application 55—76887) becomes unnecessary.

From the point of reaction yield, the present invention is also superior over the conventional methods. When the reaction solution containing sodium iodide is mixed with said hydrophilic organic solvent which can dissolve sodium iodide, the salt(s) of the desired compound of formula 1 alone becomes precipitated, the salts are removed, and the manufacture of the desired compound of a high purity and with a high yield is achieved.

The reaction is carried out by using sodium iodide as a catalyst in the aqueous solution. The reaction is preferably carried out under the following conditions: pH value, 6.0—7.5; temperature, 55°C—75°C; reaction time, 1—2 hours; and amount of sodium iodide, 10 to 50 times the equivalent weight.

Isolation of the desired substance from the obtained reaction solution is conducted preferably by the following method;

By mixing the reaction solution with said hydrophilic organic solvent which can dissolve a large excess of sodium iodide, a sodium salt of the desired subsance in solid form is obtained. Further, by repeating several times the steps of dissolving and mixing the sodium salt with the hydrophilic organic solvent, it is possible to refine the substance to a higher degree of purity. Examples of suitable organic solvents are acetone, methanol, ethanol, isopropanol, butanol, or a mixture of two or more of these solvents. If necessary, the solid thus obtained may be further refined, for instance by adsorption chromatography, ion exchange chromatography, or gel chromatography. In such a case, water or a mixture of water and the above-mentioned hydrophilic organic solvent may be used as an eluent.

The fraction containing the desired substance, i.e. a sodium salt of the compound of formula 1, is concentrated, its pH adjusted, and mixed with about 5 times the volume of hydrophilic organic solvent, and the desired sodium salt may be precipitated and obtained in a solid form. It is also possible to determine which sodium salt is obtained by controlling the pH value.

Table 1 shows a part of the result obtained from the comparison made of the acute toxicity of the sodium salt and that of the potassium salt. The table discloses that the sodium salt is superior to the latter and its low acute toxicity.

TABLE 1

Acute toxicity of 7β-[D-(-)-α-(4-carboxyimidazole-5-carboxyamido)-phenylacetamido]-3-(4-β-sulphonethyl-pyridinium)-methyl-3-cephem-4-carboxylic acid 2 metallic salt.

| | $LD_{50}$ (mouse, i.v.) |
|---|---|
| 2 Na salt | more than 3 g/kg |
| 2 K salt | 0.5—1 g/kg |

The present invention is further illustrated by the following Examples.

Example 1

16.3 Grams (30 mM) of 7β-[D-(-)-α-(4-carboxyimidazole-5-carboxyamido)-phenylacetamido]-cephalosporanic acid and 11.2 g (60 mM) of 4-pyridineethanesulphonic acid were suspended in 70 ml of water, and dissolved in 2*N* aqueous solution of sodium hydroxide by adjusting the pH value of the solution to 6.5. After adding 200 g of sodium iodide, the mixture was reacted for 70 minutes at 65°C while stirring. After the reaction liquid had been cooled, it was added dropwise to 750 ml of acetone while being ice cooled and stirred. After cooling overnight, the solid which had formed was filtered off, dissolved again in 75 ml of the water, added dropwise to 350 ml of acetone and precipitated in solid form. The solid which formed was filtered off, dissolved in 75 ml of the water, and added dropwise to 450 ml of ethanol while being ice cooled and stirred. After cooling again overnight, the solid which formed was filtered off and dried to obtain 16 g of a sodium salt of the desired compound.

The dried compound was then dissolved in 40 ml of water and its pH value was adjusted to 4. This solution was subjected to adsorption in a column which contained 700 ml of adsorbent "XAD-2" and the desired substance was eluted in 5% aqueous methanol. The fraction containing the desired substance was concentrated until the volume became 160 ml. The pH value of the solution was adjusted to 4, and the solution was then added dropwise to 800 ml of ethanol while being ice-cooled and stirred to precipitate the solid. After cooling overnight, the solid which formed was filtered off, and freeze-dried to obtain 6 g of 7β-[D-(-)-α-(4-carboxyimidazole-5-carboxyamido)-phenylacetamido]-3-(4-β-sulphoethylpyridinium)-methyl-3-cephem-4-carboxylic acid·1.5 Na salt·5 hydrate.

Elemental analysis

Measured values:

C 42.23%, H 4.43%, N 10.58%, S 7.62%, Na 4.37%

Calculated values based on $C_{28}H_{24.5}N_6O_{10}S_2Na_{1.5}\cdot5H_2O$

C 42.38%, H 4.37%, N 10.59%, S 8.08%, Na 4.35%

NMR spectrum (Solvent $D_2O$)

δ 3.20 (d.d.2H) (2 position >$CH_2$)

δ 3.45 (S.4H) (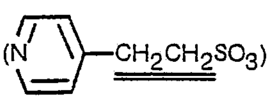)

δ 5.05 (d. 1H) (6 position — H)

δ 5.40 (d.d.2H) (3 position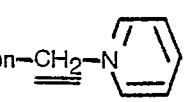

% 5.65 (S.1H) 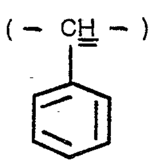

δ 5.80 (d.1H) (7 position — H)

δ 7.50 (m.5H) ( 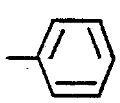 )

δ 8.00 (d.2H) (pyridine ring 3.5 position — H)

δ 8.70 (S.1H) (imidazole 2 position-H)

δ 8.85 (d.2H) (pyridine ring 2.6 position — H)

Example 2

1.1 Grams (2 mM) of 7β-[D-(-)-α-carboxyimidazole-5-carboxyamido)-p-hydroxyphenylacetamido]-cephalosporanic acid and 0.75 (4 mM) of 4-pyridineethanesulphonic acid were suspended in 10 ml of water, and dissolved in an aqueous solution of 2*N* sodium hydroxide by adjusting the pH value of the solution of 7.0. After adding 8 g of sodium iodide thereto, the resulting mixture was reacted for 2 hours at 70°C while stirring. The desired substance was then issolated and refined in a manner similar to that described in Example 1, and 0.16 g of 7β-[D-(-)-α-(4-carboxyimidazole-5-carboxyamino)-p-hydroxyphenyl-acetamido)]-3-(4-β-sulphoethylpyridinium)-methyl-3-cephem-4-carboxylic acid·2Na salt was obtained.

IR Absorbent spectrum (Nujol)

V = o (β-lactum) = 1770 $cm^{-1}$

$V_{so_2}$ ($SO_3H$) = 1230 $cm^{-1}$, 1045 $cm^{-1}$

NMR spectrum ($D_2O$)

δ 3.37 (S.4H) ( 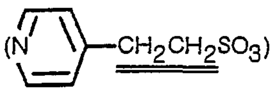 )

δ 3.47 (m.2H) (2 position >$CH_2$)

δ 5.10 (d.1H) (6 position — H)

δ 5.33 (m.2H) (3 position 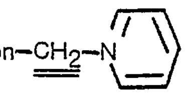 )

δ 5.58 (S.1H) 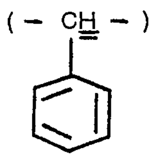

δ 5.78 (d.1H) (7 position — H)

δ 6.98 (d.2H) ( 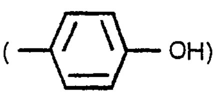 )

δ 7.48 (d.2H)

δ 7.90 (m. 3H) imidazole 2 position — H + pyridine ring 3.5 position —H

δ 8.78 (d.2H) (pyridine ring 2.6 position —H)

## Claims

1. A process for producing an imidazole dicarboxylic acid derivative of the following general formula in the form of a mono-sodium salt, the di-sodium salt, or a mixture thereof:

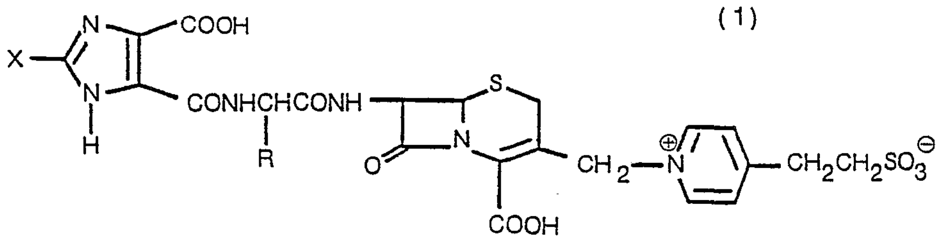

(1)

wherein each of X and R, which may be the same or different, is a hydrogen atom or a substitutent; which process comprises reacting, in an aqueous solution containing sodium iodide and sodium hydroxide, 4-pyridineethanesulphonic acid and a compound of the following general formula:—

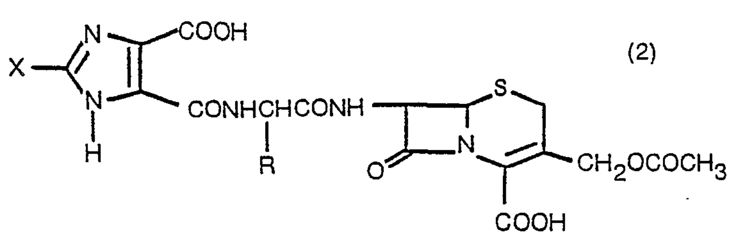

(2)

wherein X and R are as defined above; contacting the reaction solution with a hydrophilic organic solvent which can dissolve sodium iodide, said hydrophilic organic solvent comprising at least one solvent selected from alcohols containing 1 to 4 carbon atoms and acetone, thereby precipitating the said sodium salt(s) of the imidazole diacarboxylic acid derivative; and separating the said precipitated sodium salt(s).

2. A process according to claim 1, wherein pH value of the aqueous solution is in the range from 6.0 to 7.5.

3. A process according to claim 1 or 2, wherein X is a hydrogen atom, a halogen atom, a hydroxy group, a hydrocarbyloxy radical, a mercapto group, a hydrocarbylthio radical, a hydrocarbyl sulphonyl or sulphinyl radical, an amino group, a hydrocarbyl amino radical, an acylamino group, a sulphonic acid group, a nitro group, a hydrocarbyl radical or a heterocyclic radical, with any of the hydrocarbyl and heterocyclic moieties being optionally substituted; and R is a hydrogen atom, or a hydrocarbyl or heterocyclic radical which is optionally substituted.

4. A process according to claim 3, wherein X is a hydrogen atom, a halogen atom, a hydroxyl group, an alkyl-, aralkyl- or aryloxy group, a mercapto group, an alkyl-, aralkyl- or arylthio group, an alkyl-, aralkyl- or aryl- sulphonyl or -sulphinyl group, an amino group, a mono- or dialkyl-, aralkyl- or arylamino group, an acylamino group, a sulphonic acid group, a nitro group, an alkyl group, an aralkyl group, an aryl group, or a heterocyclic group, and R is a hydrogen atom, or an alkyl group, aralkyl group, aryl group or heterocyclic group; in which any of the aforementioned alkyl, aralkyl, aryl and heterocyclic groups are optionally substituted.

**Patentansprüche**

1. Verfahren zur Herstellung eines Imidazoldicarbonsäurederivats der nachstehenden allgemeinen Formel in Form eines Monoatriumsalzes, des Dinatriumsalzes oder eines Gemisches davon:

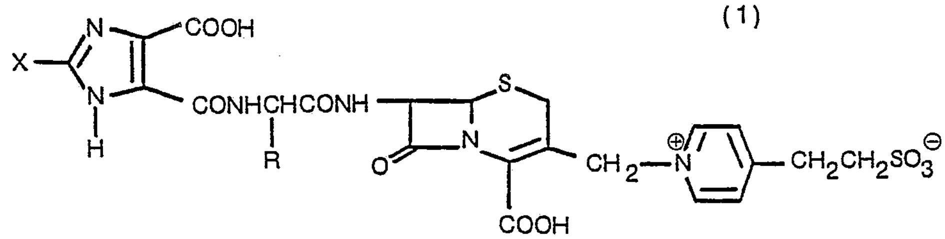

(1)

worin X und R gleich oder verschieden sein können und jeweils ein Wasserstoffatom oder einem Substituenten bedeuten;
dadurch gekennzeichnet, daß in einer Natriumiodid und Natriumhydroxid enthaltenden wässrigen Lösung 4-Pyridinethansulfonsäure und eine Verbindung der folgenden allgemeinen Formel:

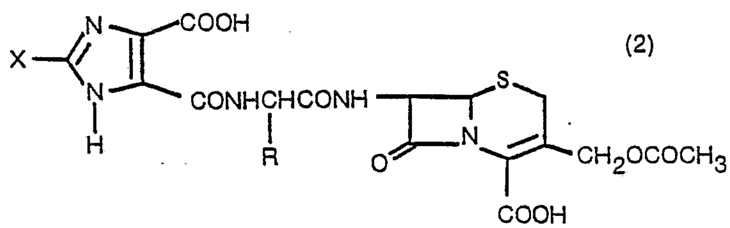

(2)

in der X und R die vorstehende Definition haben, umgesetzt werden;
die Reaktionslösung mit einem hydrophilen organischen Lösungsmittel, das Natriumiodid lösen kann, in Berührung gebracht wird, wobei das hydrophile organische Lösungsmittel mindestens ein Lösungsmittel aus der Gruppe der Alkohole mit 1 bis 4 Kohlenstoffatomen und Aceton umfaßt, um dadurch das Natriumsalz bzw. die Natriumsalze des Imidazol-dicarbonsäurederivats auszufällen; un das bzw. die so ausgefällten Natriumsalze abgetrennt werden.

2. Verfahren nach Anspruch 1, bei dem der pH—Wert der wässrigen Lösung im Bereich von 6,0 bis 7,5 liegt.

3. Verfahren nach Anspruch 1 oder 2, worin X ein Wasserstoffatom, Halogenatom, eine Hydroxygruppe, einen Kohlenwasserstoffoxyrest, eine Mercaptogruppe, einen Kohlenwasserstoffthiorest, einen Kohlenwasserstoffsulfonyl- oder -sulfinyl-Rest, eine Aminogruppe, einen Kohlenwasserstoff-amino-Rest, eine Acylaminogruppe, eine Sulfonsäuregruppe, Nitrogruppe, einen Kohlenwasserstoffrest oder einen heterocyclischen Rest bedeutet, wobei die Kohlenwasserstoffreste und heterocyclischen Reste gegebenenfalls substituiert sind; und R für ein Wasserstoffatom oder einen Kohlenwasserstoff- oder heterocyclischen Rest, der gegebenenfalls substituiert ist, steht.

4. Verfahren nach Anspruch 3, worin X ein Wasserstoffatom, Halogenatom, eine Hydroxygruppe, eine Alkyl-, Aralkyl- oder Aryloxygruppe, eine Mercaptogruppe, eine Alkyl-, Aralkyl- oder Arylthiogruppe, eine

Alkyl-, Aralkyl, oder Aryl-sulfonyl- oder -sulfinyl-Gruppe, eine Aminogruppe, eine Mono- oder Dialkyl-Aralkyl- oder Arylamino-Gruppe, eine Acylaminogruppe, eine Sulfonsäuregruppe, eine Nitrogruppe, Alkylgruppe, Aralkylgruppe, Arylgruppe oder eine heterocyclische Gruppe bedeutet, und R ein Wasserstoffatom oder eine Alkylgruppe, Aralkylgruppe, Arylgruppe oder heterocyclische Gruppe ist, wobei jede beliebige der vorstehend erwähnten Alkyl-, Aralkyl-, Aryl- und heterocyclischen Gruppen gegebenenfalls substituiert ist.

**Revendications**

1. Procédé pour la production d'un dérivé d'acide imidazoledicarboxylique de formule générale suivante sous la forme d'un sel monosodique, du sel disodique, ou de leur mélange:

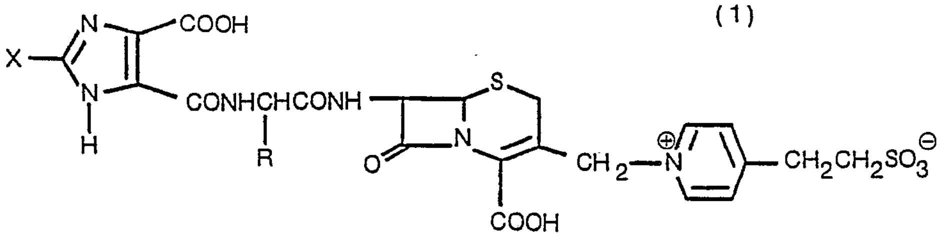
(1)

dans laquelle X et R, qui peuvent être identiques ou différents, représentent chacun un atome d'hydrogène ou un substituant; ledit procédé consistant à faire réagir, dans une solution aqueuse contenant de l'iodure de sodium et de l'hydroxyde de sodium, l'acide 4-pyridineéthanesulfonique et un composé de formule générale suivante:

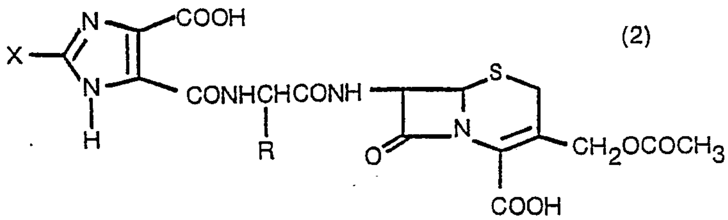
(2)

dans laquelle X et R sont comme définis ci-dessus; à mettre en contact la solution de réaction avec un solvant organique hydrophile qui peut dissoudre l'iodure de sodium, ledit solvant organique hydrophile comprenant au moins un solvant choisi parmi les alcools en $C_1$—$C_4$et l'acétone, de manière à précipiter le(s)dit(s) sel(s) de sodium du dérivé d'acide imidazoledicarboxylique; et à séparer le(s)dit(s) sel(s) de sodium précipité(s).

2. Procédé selon la revendication 1, caractérisé en ce que le pH de la solution aqueuse est dans la gamme de 6,0 à 7,5.

3. Procédé selon la revendication 1 ou 2, caractérisé en ce que X est un atome d'hydrogène ou d'halogène ou un groupe hydroxy, un radical hydrocarbyloxy, un groupe mercapto, un radical hydrocarbylthio, le radical hydrocarbylsulfonyle ou -sulfinyle, un groupe amino, un radical hydrocarbylamino, un groupe acylamino, un groupe acide sulfonique, un groupe nitro, un radical hydrocarbyle ou un radical hétérocyclique, l'un quelconque des restes hydrocarbyles et hétérocycliques étant facultativement substitué; et R est un atome d'hydrogène ou un radical hydrocarbyle ou hétérocyclique qui est facultativement substitué

4. Procédé selon la revendication 3, caractérisé en ce que X est un atome d'hydrogène ou d'halogène ou un groupe hydroxyle, un groupe alkyl-, aralkyl- ou aryloxy, un groupe mercapto, un groupe alkyl-, aralkyl- ou arylthio, un groupe alkyl-, aralkyl- ou aryl- sulfonyle ou -sulfinyle, un groupe amino, un groupe mono- ou dialkyl-, aralkyl- ou arylamino, un groupe acylamino, un groupe acide sulfonique, un groupe nitro, un groupe alkyle, un groupe aralkyle, un groupe aryle ou un groupe hétérocyclique; et R est un atome d'hydrogène ou un groupe alkyle, un groupe aralkyle, un groupe aryle ou un groupe hétérocyclique; dans lesquels l'un quelconque des groupes alkyles, aralkyles, aryles et hétérocycliques mentionnés ci-dessus est facultativement substitué.